# EUROPEAN PATENT APPLICATION

(11) **EP 2 437 189 A2**
(43) Date of publication of application: **04.04.2012**
(21) Application number: 11182991.7
(22) Date of filing: 27.09.2011
(51) Int. Cl.: G06F 19/00

(54) **Healthcare information system**

(30) Priority: 27.09.2010 JP 2010215822
(71) Applicant: Kabushiki Kaisha Toshiba, Tokyo 105-8001 (JP); Toshiba Medical Systems Corporation, Otawara-shi Toshigi 324-8550 (JP)
(72) Inventor: Konishi, Hayato, 324-8550, Tochigi Otawara-shi (JP); Sakaue, Kousuke, 324-8550, Tochigi Otawara-shi (JP); Yagi, Minoru, 324-8550, Tochigi Otawara-shi (JP)
(74) Representative: Granleese, Rhian Jane

(57) **Abstract**

According to one embodiment, a healthcare information system includes a measurement type storage unit (32), input unit (31), measurement type determination unit (33), acquisition unit (36), and measurement value storage unit (32). The measurement type storage unit (32) stores a plurality of measurement types and healthcare information in association with each other. The input unit (31) inputs healthcare information associated with a monitoring object person. The measurement type determination unit (33) determines a measurement type associated with the input healthcare information in the measurement type storage unit (32). The acquisition unit (36) acquires a measurement value of the monitoring object person associated with the determined measurement type via a healthcare device (1). The measurement value storage unit (32) stores the acquired measurement value.

## Description

### FIELD

Embodiments described herein relate generally to a healthcare information system.

### BACKGROUND

Constant healthcare monitoring techniques are expected to contribute to early diagnosis, disease prevention, and health maintenance. Some diseases and abnormalities require long-term monitoring over several years to several ten years. In a constant healthcare monitoring environment, many healthcare devices always acquire the healthcare information of a monitoring object person, and a database stores the acquired healthcare information. In consideration of convenience, economical reasons, and the like, it is practically difficult for a user himself/herself to own and manage a large-capacity storage medium for storing an enormous data amount of healthcare information. Therefore, the monitoring efficiency is low.

### BRIEF DESCRIPTION OF THE DRAWINGS

FIG. 1 is a view showing the concept of a healthcare information system according to the first embodiment;
FIG. 2 is a block diagram showing the arrangement of the healthcare information system according to the first embodiment;
FIG. 3 is a view showing a concrete example of disease data input by a disease data input unit in FIG. 2;
FIG. 4 is a view showing an example of a monitoring rule table stored in a terminal database in FIG. 2;
FIG. 5 is a view showing another example of the monitoring rule table stored in the terminal database in FIG. 2;
FIG. 6 is a view showing still another example of the monitoring rule table stored in the terminal database in FIG. 2;
FIG. 7 is a flowchart showing a typical procedure for monitoring rule determination processing performed by a monitoring rule determination unit in FIG. 2;
FIG. 8 is a block diagram showing a concrete example of monitoring rule determination processing performed by the monitoring rule determination unit in FIG. 2;
FIG. 9 is a block diagram showing the arrangement of a healthcare information system according to the second embodiment;
FIG. 10 is a view showing a concrete example of a communication setting file set by a communication setting unit in FIG. 9;
FIG. 11 is a view showing a concrete example of an object person file set by a'monitoring object person setting unit in FIG. 9;
FIG. 12 is a view showing an outline of a healthcare information system according to the third embodiment;
FIG. 13 is a block diagram showing the arrangement of the healthcare information system according to the third embodiment;
FIG. 14 is a block diagram showing a concrete example of monitoring target disease extraction processing by a disease data extraction unit in FIG. 13;
FIG. 15 is a view showing an outline of a healthcare information system according to the fourth embodiment;
FIG. 16 is a view showing the arrangement of the healthcare information system according to the fourth embodiment;
FIG. 17 is a view showing a concrete example of reference data acquisition processing by a reference data acquisition unit in FIG. 16; and
FIG. 18 is a block diagram showing the arrangement of a healthcare information system according to a modification.

### DETAILED DESCRIPTION

In general, according to one embodiment, a healthcare information system includes a measurement type storage unit, input unit, measurement type determination unit, acquisition unit, and measurement value storage unit. The measurement type storage unit stores a plurality of measurement types and healthcare information in association with each other. The input unit inputs healthcare information associated with a monitoring object person. The measurement type determination unit determines a measurement type associated with the input healthcare information in the measurement type storage unit. The acquisition unit acquires a measurement value of the monitoring object person associated with the determined measurement type via a healthcare device. The measurement value storage unit stores the acquired measurement value.

Healthcare information according to this embodiment includes at least one of patient information, examination information, medical interview information, and disease information. Patient information includes, for example, a birth date, age, sex, medical history, allergy information, family information, and family medical history. Examination information includes, for example, information such as anthropometric measurement values, blood test results, examination results obtained by various kinds of image diagnostic apparatuses, and measurement values obtained by healthcare devices. Examination information may also include threshold information indicating the degrees of abnormality of these measurement values and the respective examination items. Medical interview information includes medical interview results obtained by an interviewing doctor, such as a symptom, disease name, and lifestyle habit. Disease information includes information such as disease names, findings, and past histories of them.

A healthcare information system according to this embodiment will be described below with reference to the accompanying drawing. For the sake of a concrete description, assume that healthcare information according to this embodiment is a disease name, unless otherwise specified.

### (First Embodiment)

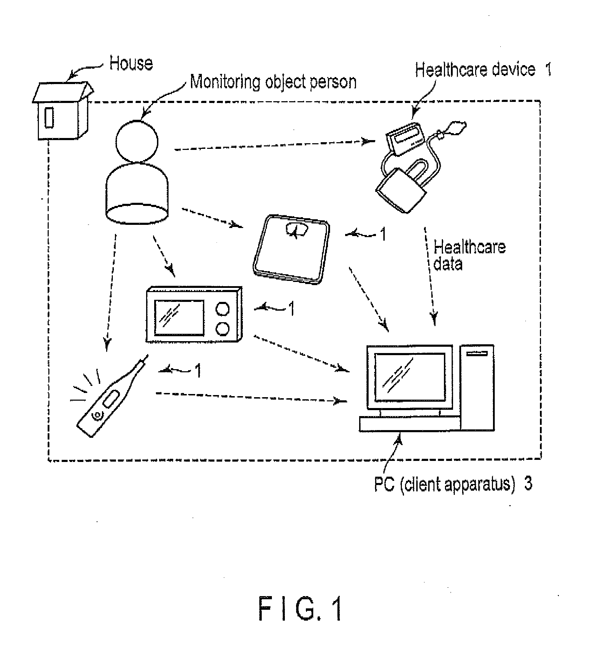

FIG. 1 is a view showing the concept of a healthcare information system according to the first embodiment. As shown in FIG. 1, the healthcare information system according to the first embodiment is typically designed within a house or the like. A monitoring object person measures a health index with a healthcare device 1. The healthcare device 1 as a general device is, for example, a thermometer which measures the body temperature, a pulsimeter which measures the pulse, a hemodynamometer which measures the blood pressure, or a weight scale which measures the body weight. A PC (Personal Computer) 3 in the healthcare information system stores and manages the data of the measurement values of health index such as body temperature, pulse, blood pressure, and body weight. The data of the measurement value of a health index will be referred to as healthcare data hereinafter. The monitoring object person monitors health indexes corresponding to the diseases that the person has by using the PC 3. If, for example, the monitoring object person suffers from high blood pressure, the person can monitor a health index of the blood pressure measured by a hemodynamometer with the PC 3. Note that the place where the healthcare information system is constructed is not limited to the inside of a house. The healthcare information system can be constructed in any place where the healthcare device 1 and the PC 3 which manages it exist.

The PC 3 is connected to a server apparatus (not shown in FIG. 1) via a network. Alternatively, data may be written from the PC 3 to a storage medium, a memory card or the like, and the server apparatus may read the written data. The server apparatus is typically installed in a hospital or the like and manages medical data associated with a plurality of patients. The server apparatus is also connected to a healthcare information system constructed in a hospital or the like. The PC 3 transmits healthcare data acquired via the healthcare device 1 to the server apparatus via the network. The server apparatus receives and sores the transmitted healthcare data as medical data. In this manner, the PC 3 functions as a client terminal. The PC 3 will be referred to as a client apparatus hereinafter.

Note that the PC 3 is not limited to a desktop computer like that shown in FIG. 1, and may be a computer such as a note personal computer or a PDA (Personal Digital Assistant).

The healthcare device 1 according to this embodiment is wiredly or wirelessly connected to the PC 3. The healthcare device 1 can automatically measure healthcare data and supply the measured healthcare data to the PC 3 wiredly or wirelessly. The healthcare device 1 may perform this operation in response to a request made by the PC 3. The number of healthcare devices 1 connected to the client apparatus 3 is not limited to one but may be plural. The healthcare device 1 may be of a type that can measure only one type of healthcare data or a type that can measure a plurality of types of healthcare data.

As the healthcare device 1 capable of automatically measuring healthcare data, for example, there is known a watch-type composite healthcare sensor, ring-type pulse oximeter, sensor-equipped GPS shoes, oculomotor sensor, brain wave sensor, pulse sensor, or blood pressure sensor. A watch-type composite healthcare sensor is worn on a wrist of the monitoring object person to automatically measure the body movement, electric conductivity, and cutaneous temperature. A ring-type pulse oximeter is worn on a finger of the monitoring object person to automatically measure the concentration or amount of oxygen in blood. Sensor-equipped GPS shoes are shoes which are equipped with a GPS and a pressure sensor to automatically measure the position and foot pressure of the monitoring object person. An oculomotor sensor is built in eyeglasses or goggles to automatically measure the momentum and line-of-sight direction of an eyeball. A brain wave sensor is worn on the head portion of the monitoring object person to automatically measure the brain wave. A pulse sensor is worn on a finger of the monitoring object person to automatically measure the pulse. A blood pressure sensor is built in, for example, a toilet seat to automatically measure the blood pressure. Note that the healthcare device 1 according to this embodiment is not limited to those described above. As the healthcare device 1 according to the embodiment, it is possible to use any type of device as long as it can transmit healthcare data to the client apparatus 3 wiredly or wirelessly.

According to the above description, the healthcare information system is installed in a house. However, the healthcare information system according to this embodiment may be installed in a patient's room in a hospital. In this case, the client apparatus 3 is provided in the patient's room in the hospital.

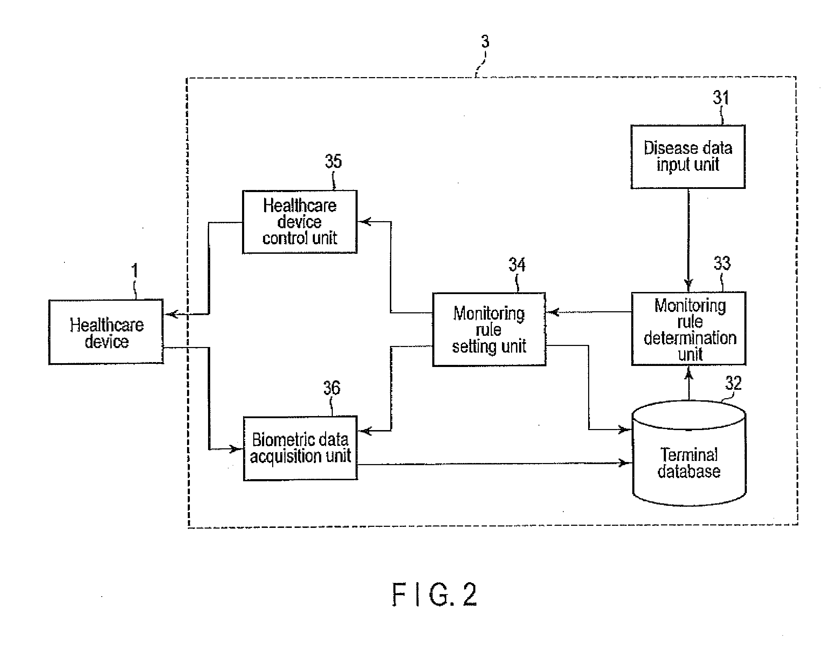

FIG. 2 is a block diagram showing the arrangement of the healthcare information system according to the first embodiment. As shown in FIG. 2, the healthcare information system includes the healthcare device 1 and the client apparatus 3. The healthcare device 1 is connected to the client apparatus 3 either wiredly or wirelessly. Instead, the healthcare device 1 may be configured to read data from a storage medium, a memory card, or the like. The client apparatus 3 includes a disease data input unit 31, a terminal database 32, a monitoring rule determination unit 33, a monitoring rule setting unit 34, a healthcare device control unit 35, and a healthcare data acquisition unit 36.

The disease data input unit 31 inputs disease data associated with the monitoring object person. Disease data includes, for example, disease name data. The disease data may also include the data of severity such as a stage, in addition to the disease name data. The disease data input unit 31 may directly input the identifier of disease data via a GUI or the like or may input a file, table, or the like in which disease data is recorded. The disease data input unit 31 may input disease data from a healthcare information system such as an electronic medical record system via a network. Note that the disease data input unit 31 may input data associated with diseases such as complications and possible intercurrent diseases as disease data as well as data such as the diseases which the monitoring object person has and their stages.

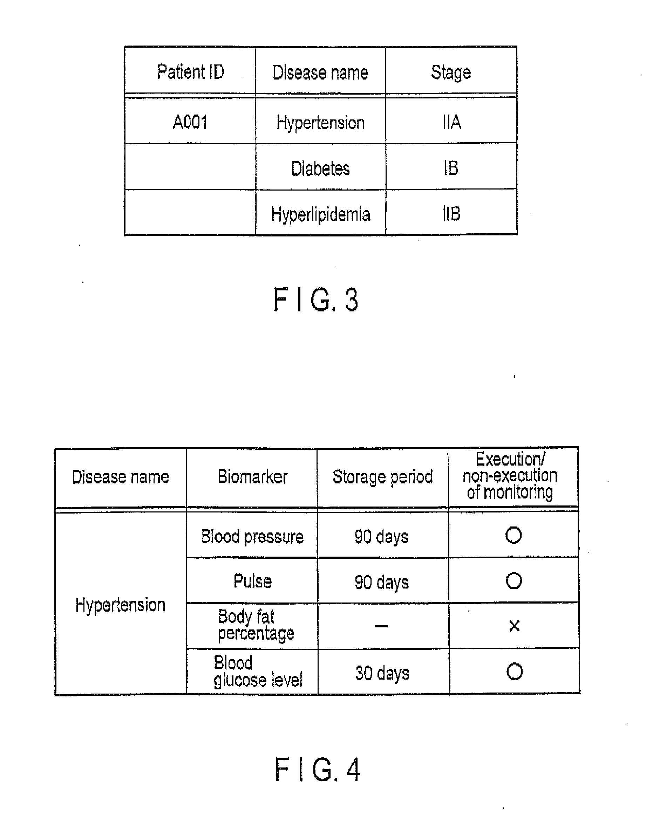

FIG. 3 is a view showing a concrete example of disease data input by the disease data input unit 31. As shown in FIG. 3, the disease data includes the patient ID of the monitoring object person, a disease name, and a stage. In this case, the patient ID of the monitoring object person is "A001", and the patient suffers from "hypertension" of stage "IIA", "diabetes" of stage "IB", and "hyperlipidemia" of stage "IIB".

The terminal database 32 stores a plurality of disease names and a plurality of healthcare data conditions in association with each other. The healthcare data conditions include at least one of a measurement type and a healthcare device type. The terminal database 32 stores disease name data and measurement type data. A measurement type indicates a health index name or its identification number. In this manner, the terminal database 32 functions as a measurement type storage unit which stores a plurality of disease names and a plurality of measurement types in association with each other. Note that the terminal database 32 stores measurement types and the types of healthcare devices 1 in association with each other. Since one healthcare device does not always measure only one measurement type as in the above case, measurement types and healthcare device types may have a one-to-one correspondence or a many-to-one correspondence. In addition, since a plurality of healthcare devices measure one measurement type, measurement types and healthcare device types may have a one-to-many correspondence.

The terminal database 32 stores a plurality of healthcare data conditions and a plurality of monitoring rules in association with each other. The monitoring rules include at least one of the storage period of healthcare data and an acquisition timing. The terminal database 32 stores storage period data and acquisition timing data. A storage period is set in accordance with each measurement type. A storage period is determined in accordance with a medical characteristic corresponding to a measurement type. An acquisition timing is set in accordance with each measurement type. An acquisition timing is determined in accordance with a medical characteristic corresponding to a measurement type.

Healthcare data monitoring rules include a combination of a measurement type, healthcare device type, storage period, acquisition timing, and execution/non-execution of monitoring. Typically, the terminal database 32 holds a table (to be referred to as a monitoring rule table hereinafter) which associates a plurality of disease names with a plurality of monitoring rules.

FIG. 4 is a view showing an example of a monitoring rule table. The monitoring rule table shown in FIG. 4 includes a disease name item, health index name, storage period item, and monitoring execution/non-execution item. That is, this table indicates, in association with each disease name, a health index name, information indicating whether to monitor the health index, and the storage period of the healthcare data of the health index. For example, in the case of hypertension, the table indicates that a blood pressure, pulse, and blood glucose level are monitored, the storage period of blood pressure data is 90 days, the storage period of pulse data is 90 days, and the storage period of blood glucose level data is 30 days.

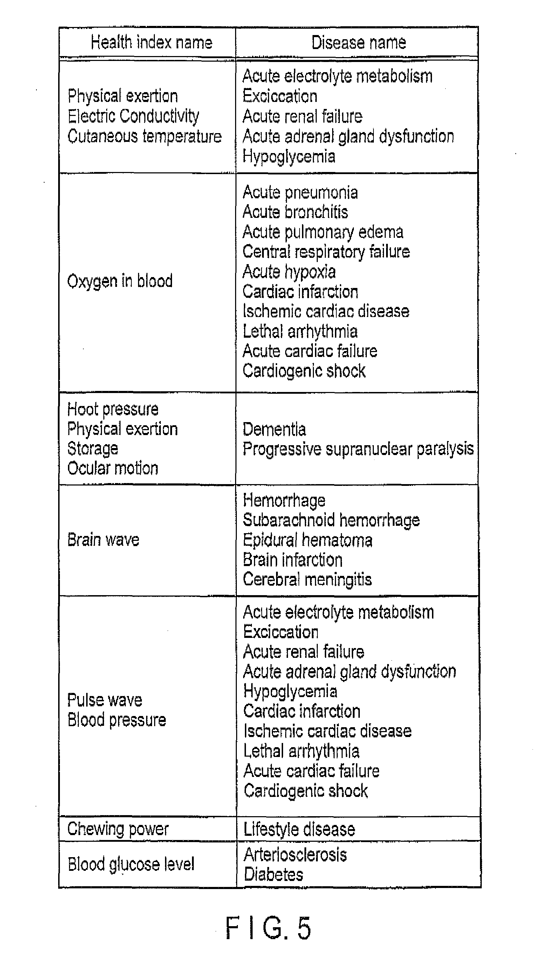

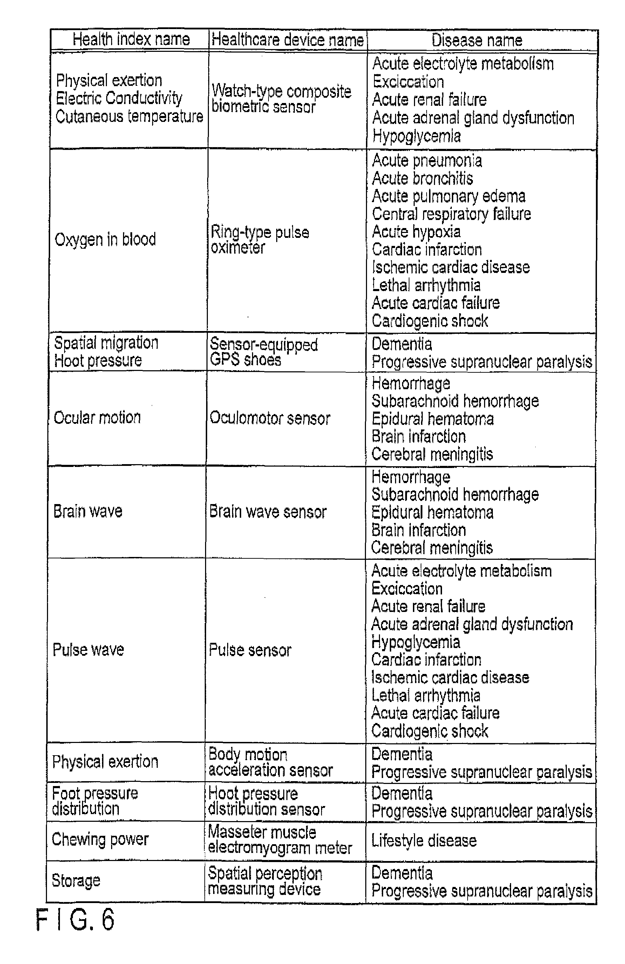

Note that the contents of the monitoring rule table are not limited to the example described above. As shown in FIG. 5, combinations of health index names and disease names set in the monitoring rule table include various examples. As shown in FIG. 6, it is possible to associate health index names, disease names, and healthcare device names with each other.

The terminal database 32 further stores various data and tables in addition to disease name data and monitoring rule data. For example, the terminal database 32 stores the healthcare data acquired by the healthcare data acquisition unit 36 via the healthcare device 1. In this manner, the terminal database 32 functions as a storage unit which stores healthcare data. The terminal database 32 stores healthcare data only for the above storage periods. The terminal database 32 manages a communication setting file associated with the healthcare device 1. The terminal database 32 functions as a communication setting management unit which manages a communication setting file. The terminal database 32 also manages an object person file associated with the monitoring object person. In this manner, the terminal database 32 also functions as an object person management unit which manages the object person file. The communication setting file and the object person file will be described later.

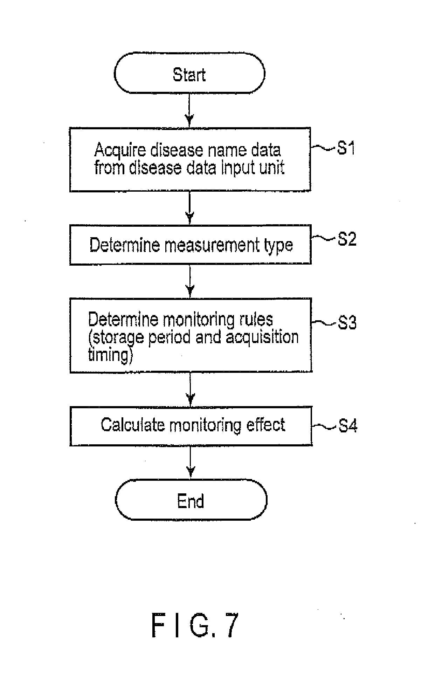

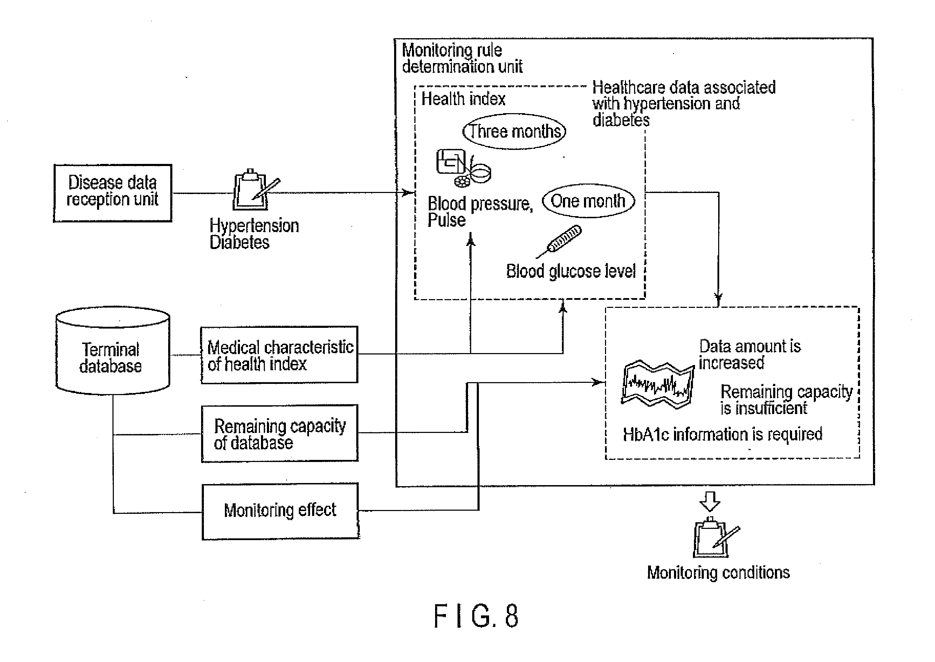

The monitoring rule determination unit 33 determines monitoring rules by using the monitoring rule table based on the disease name in the disease data input by the disease data input unit 31. FIG. 7 is a flowchart showing a typical procedure for monitoring rule determination processing performed by the monitoring rule determination unit 33. FIG. 8 is a view showing a concrete example of monitoring rule determination processing. Assume that, referring to FIG. 8, the disease names as monitoring targets are hypertension and diabetes. Assume also that the monitoring object person has already started monitoring health indexes by using the healthcare information system. That is, assume that the terminal database 32 has stored the healthcare data of hypertension and diabetes measured within a proper periods.

As shown in FIG. 7, when disease name data is input to the disease data input unit 31, the monitoring rule determination unit 33 acquires the disease name data from the disease data input unit 31 (step S1). If the monitoring object person is an unhealthy body, disease name data written in findings, diagnosis results, and the like is acquired. If the monitoring object person is a healthy body, the data of disease names checked in medical examination or the like is acquired. Assume that the latest disease name data is periodically input to the disease data input unit 31 to determine a monitoring rule. In the case shown in FIG. 8, "hypertension" and "diabetes" which are disease names as monitoring targets are acquired.

Upon acquiring the disease name data, the monitoring rule determination unit 33 determines a measurement type associated with the acquired disease name data (step S2). More specifically, the monitoring rule determination unit 33 searches the monitoring rule table by using a disease name as a keyword, and determines a healthcare data condition associated with the disease name on the monitoring rule table. That is, the monitoring rule determination unit 33 determines a measurement type and healthcare device type in accordance with the disease name. In this manner, the monitoring rule determination unit 33 functions as a measurement type determination function. In the case shown in FIG. 8, the monitoring rule determination unit 33 determines a blood pressure, pulse, and blood glucose level as measurement types by using the monitoring rule table. Note that the determined measurement types are set to "execution" of monitoring in the monitoring execution/non-execution item in the monitoring rule table.

Upon determining the measurement types, the monitoring rule determination unit 33 determines monitoring rules associated with the determined health indexes (step S3). More specifically, the monitoring rule determination unit 33 determines monitoring rules associated with the determined health indexes in accordance with the medical characteristics of the health indexes corresponding to the determined measurement types. That is, the monitoring rule determination unit 33 determines the storage periods and acquisition timings of the determined healthcare data. Typically, the monitoring rule determination unit 33 determines monitoring rules by using the monitoring rule table. As described above, the monitoring rule determination unit 33 has a monitoring rule determination function. For example, the blood pressure always varies. It is therefore preferable to measure the blood pressure at the same time every time over a long period. It is preferable to calculate a monitoring effect by using the average value of the blood pressures measured at the same time over a long period. Therefore, the storage period of this data is set to a long period, for example, three months. Acquisition timings are set to the same times, e.g., at the wake-up time, before breakfast, and before bedtime, under the same conditions. The blood glucose level before eating greatly differs from that after eating. In addition, blood glucose level data for one month are required to check the time course of blood glucose levels. The storage period of this data is therefore set to an intermediate period, e.g., one month. Acquisition timings are respectively set to a time before eating and a time after eating. The storage period of such data may be determined in accordance with the storage period of each measurement type determined by a doctor or a healthcare information system and the symptom of the monitoring object person. Alternatively, the monitoring rule determination unit 33 may determine a storage period in accordance with the remaining capacity of the terminal database 32 without using the monitoring rule table. In addition, it is possible to set, as an acquisition timing, a measurement frequency indicating the number of times of measurement in a predetermined period. A measurement frequency is, for example, two times a day or seven times a week. If the remaining capacity of the terminal database 32 is not sufficient, healthcare data in the terminal database 32 is deleted.

Upon determining monitoring rules, the monitoring rule determination unit 33 calculates monitoring effects based on the healthcare data stored in the terminal database 32 (step S4). The monitoring results include the progress of treatment and the transitions of health index values. The monitoring effects are displayed on the display of the PC and stored in the terminal database 32. Observing the monitoring effects allows the monitoring object person to check whether, for example, the treatment shows good progress, the drugs work properly, and the health index measurement values change in a favorable way. This makes it possible for the person to manage his/her health on the PC at home. In some cases, upon observing monitoring effects, the operator determines that some health index under monitoring is not suitable for monitoring or need not be monitored after the completion of treatment. In this case, the monitoring rule determination unit 33 can exclude this health index from the monitoring targets in accordance with the operation of setting execution/non-execution of monitoring by the operator via an input device such as the client apparatus 3. The monitoring rule determination unit 33 can also add a new health index to the monitoring targets in accordance with the operation of setting execution/non-execution of monitoring by the operator via an input device such as the client apparatus 3. For example, in the case shown in FIG. 8, HbAlc is added as a new monitoring target.

Upon completion of step S4, the monitoring rule determination unit 33 finishes the monitoring rule determination processing.

The monitor of the client apparatus 3 preferably displays monitoring rules in accordance with an instruction from the user. This allows the monitoring object person to check the monitoring rules. In some cases, the monitoring object person determines that there is a problem in the monitoring rules. The monitoring rule determination unit 33 can change the monitoring rules in accordance with an instruction from the user, as needed.

As shown in FIG. 2, the monitoring rule setting unit 34 sets the monitoring rules determined by the monitoring rule determination unit 33 in the terminal database 32. The terminal database 32 manages the set monitoring rules.

The healthcare device control unit 35 controls the healthcare device 1. The healthcare device 1 measures healthcare data associated with the monitoring object person under the control of the healthcare device control unit 35. The healthcare device control unit 35 performs data communication with the healthcare device 1 by using the communication setting file stored in the terminal database 32. The healthcare data acquisition unit 36 acquires healthcare data associated with the monitoring object person via the healthcare device 1. The types of acquired healthcare data correspond to the measurement types determined by the monitoring rule determination unit 33. The terminal database 32 stores the acquired healthcare data.

As shown in FIG. 2, the monitoring rule setting unit 34 sets the monitoring rules determined by the monitoring rule setting unit 34 in the healthcare device control unit 35 or the healthcare data acquisition unit 36.

Healthcare data acquisition processing will be described separately in the case in which the monitoring rules are set in the healthcare data acquisition unit 36 (healthcare data acquisition method 1) and in the case in which the monitoring rules are set in the healthcare device control unit 35 (healthcare data acquisition method 2).

### [Healthcare data Acquisition Method 1]

When the monitoring rules are set in the healthcare data acquisition unit 36, the healthcare device control unit 35 controls the healthcare device 1 to always measure healthcare data regardless of the monitoring rules. Note that if the healthcare device 1 can always measure healthcare data by itself without any control from the healthcare device control unit 35, the healthcare device control unit 35 is not necessary.

The healthcare device 1 always measures the healthcare data of the monitoring object person. The measured healthcare data is supplied to the healthcare data acquisition unit 36. In the case of acquisition method 1, the healthcare device 1 measures not only healthcare data associated with health indexes as monitoring targets but also healthcare data associated with health indexes which are not monitoring targets, and supplies all the healthcare data to the healthcare data acquisition unit 36. The healthcare device 1 measures healthcare data both in the storage period determined by the monitoring rule determination unit 33 and a period other than the storage period, and supplies all the healthcare data to the healthcare data acquisition unit 36. The healthcare device 1 measures healthcare data both inside the acquisition timing determined by the monitoring rule determination unit 33 and outside the acquisition timing, and supplies all the healthcare data to the healthcare data acquisition unit 36.

The healthcare data acquisition unit 36 acquires data, of the healthcare data from the healthcare device 1, which matches the monitoring rules determined by the monitoring rule determination unit 33. If, for example, the disease name as a monitoring target is "blood glucose level", the storage period is "three months", and the acquisition timing is "8:00 every day", the healthcare data acquisition unit 36 acquires healthcare data matching this disease name and the monitoring rules, and discards other healthcare data. The terminal database 32 stores the acquired healthcare data.

### [healthcare information Acquisition Method 2]

When the monitoring rules are set in the healthcare device control unit 35, the healthcare device control unit 35 controls the healthcare device 1 to measure healthcare data corresponding to the set monitoring rules. More specifically, the healthcare device control unit 35 controls the healthcare device 1 corresponding to the measurement type as a monitoring target for the measurement of healthcare data associated with the health index as the monitoring target. In this case, the healthcare device control unit 35 controls the healthcare device 1 to measure healthcare data in the storage period and at the acquisition timing as monitoring rules.

The healthcare device 1 measures healthcare data under the control of the healthcare device control unit 35. The measured healthcare data is supplied to the healthcare data acquisition unit 36. If, for example, the disease name as a monitoring target is "blood glucose level", the storage period is "three months", and the acquisition timing is "8:00 every day", the healthcare device 1 for blood glucose level (for example, a blood glucose level meter) automatically measures the blood glucose level at 8:00 every day over three months, and supplies the measured blood glucose level data to the healthcare data acquisition unit 36.

The healthcare data acquisition unit 36 acquires the healthcare data from the healthcare device 1. The terminal database 32 stores the acquired healthcare data.

With the above arrangement, the healthcare information system according to the first embodiment can determine a measurement type in accordance with a disease as a monitoring target, and cause the terminal database 32 to store only the healthcare data associated with the determined measurement type. Therefore, the data amount of healthcare data stored in the terminal database 32 decreases as compared with the prior art in which healthcare data which do not match monitoring rules are stored. As the data amount decreases in this manner, it is possible to suppress the data capacity of the terminal database 32 in the healthcare device 1. In addition, it is possible to reduce the complexity of data management.

In this manner, the healthcare information system according to the first embodiment can achieve an improvement in monitoring efficiency.

Note that the client apparatus 3 is assumed to be a desktop computer, note personal computer, or PDA, but is not limited to them. The client apparatus 3 according to this embodiment can be applied to any information device having a communication function, computation function, and storage function, e.g., a cellular phone, portable music player, or electronic book player (electronic book reader device).

### (Second Embodiment)

When monitoring healthcare data, it is necessary first to make communication setting for a healthcare device 1 which measures healthcare data, setting for a monitoring object person, and the like. A healthcare information system according to the second embodiment further includes an arrangement which allows to make communication setting for the healthcare device 1 and setting for a monitoring object person. The healthcare information system according to the second embodiment will be described below. Note that the same reference numerals in the following description denote constituent elements having almost the same functions as those in the first embodiment, and a repetitive description will be made only when required.

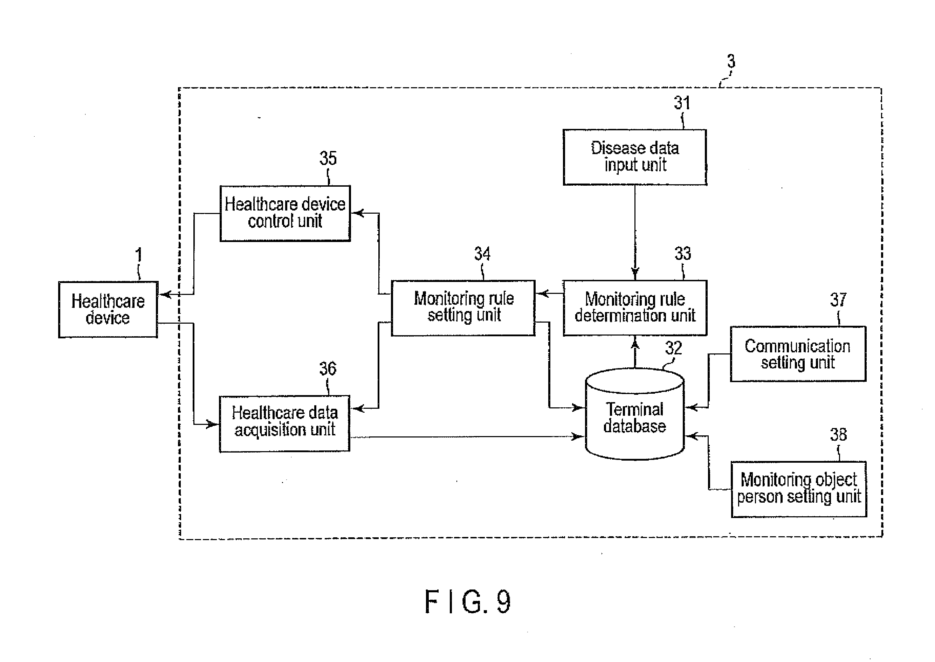

FIG. 9 is a block diagram showing the arrangement of the healthcare information system according to the second embodiment. As shown in FIG. 9, a client apparatus 3 according to'the second embodiment further includes a communication setting unit 37 and a monitoring object person setting unit 38.

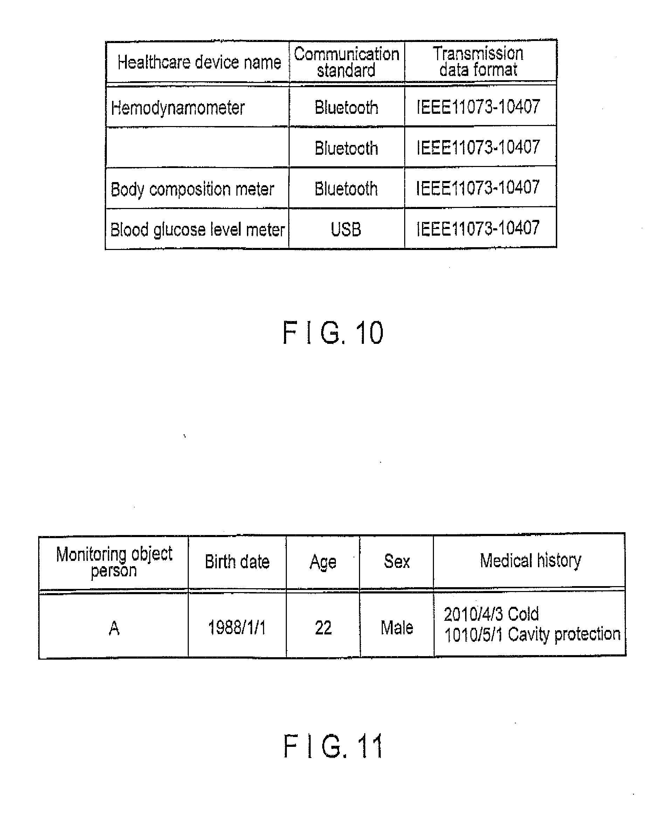

The communication setting unit 37 receives a communication setting file from the new healthcare device 1, and sets a communication setting file for the healthcare device 1 in a terminal database 32 in accordance with the received communication setting file. Setting the communication setting file in the terminal database 32 will complete communication setting for the new healthcare device 1. The communication setting file includes at least one communication setting data including the transmission data format and communication standard of the healthcare device 1. FIG. 10 is a view showing a concrete example of a communication setting file. As shown in FIG. 10, communication standards include, for example, Bluetoath^{®} and USB (Universal Serial Bus). Note that the communication standards are not limited to them. For example, as communication standards according to this embodiment, it is possible to set any existing communication standards such as Continua which is a connection standard for healthcare devices. For example, IEEE11073-10407 is set as a transmission data format. Note that the transmission data format to be set is not limited to this. It is possible to set any other existing transmission data format as a transmission data format according to this embodiment. Note that the communication setting data included in the communication setting file is not limited to only the above communication standard and transmission data format. For example, the communication setting data may include any kinds of data that allow identification of a healthcare device, e.g., the manufacturer of the healthcare device, the name of the healthcare device, and the product serial number. A method of receiving a communication setting file may be a method of making an operator such as an monitoring object person directly input data via the input device of the client apparatus 3 or a method of inputting a file and data from another apparatus.

As described above, the communication setting unit 37 allows to set or add a healthcare device, and allows to monitor healthcare data using a new healthcare device.

The monitoring object person setting unit 38 receives the object person file of a new monitoring object person, and sets the object person file of the new monitoring object person in the terminal database 32 in accordance with the received object person file. Setting the object person file in the terminal database 32 will complete setting for the new monitoring object person. The object person file includes at least one of the age, sex, and medical history of the monitoring object person. FIG. 11 is a view showing a concrete example of an object person file. As shown in FIG. 11, the medical history is a history of diseases that the person had in the past. In the case shown in FIG. 11, referring to the medical history, a monitoring object person A visited a hospital for treatment for a cold April 3, 2010, and also visited a hospital for cavity protection May 1, 2010. Note that the object person data included in the object person file is not limited to only the above age, sex, and medical history. For example, the object person data may include any kinds of data associated with the monitoring object person, e.g., the birth date and name of the monitoring object person, allergy information, and diagnosis results. A method of receiving an object person file may be a method of making an operator such as an monitoring object person directly input data via the input device of a PC or a method of inputting a file and data from another apparatus.

As described above, the monitoring object person setting unit 38 allows to set or add a monitoring object person and monitor healthcare data associated with the new monitoring object person.

In this manner, the healthcare information system according to the second embodiment can achieve an improvement in monitoring efficiency.

### (Third Embodiment)

The diseases that a monitoring object person has may include a serious disease (to be referred to as a monitoring target disease hereinafter) of which the monitoring object person needs to make early diagnosis or which requires care on prognosis. A monitoring target disease should be monitored preferentially over other non-serious diseases (to be referred to as non-monitoring target diseases hereinafter). The healthcare information system according to the third embodiment achieves an improvement in monitoring efficiency by classifying diseases into monitoring target diseases and non-monitoring target diseases. The healthcare information system according to the third embodiment will be described below. Note that the same reference numerals in the following description denote constituent elements having almost the same functions as those in the first and second embodiments, and a repetitive description will be made only when required.

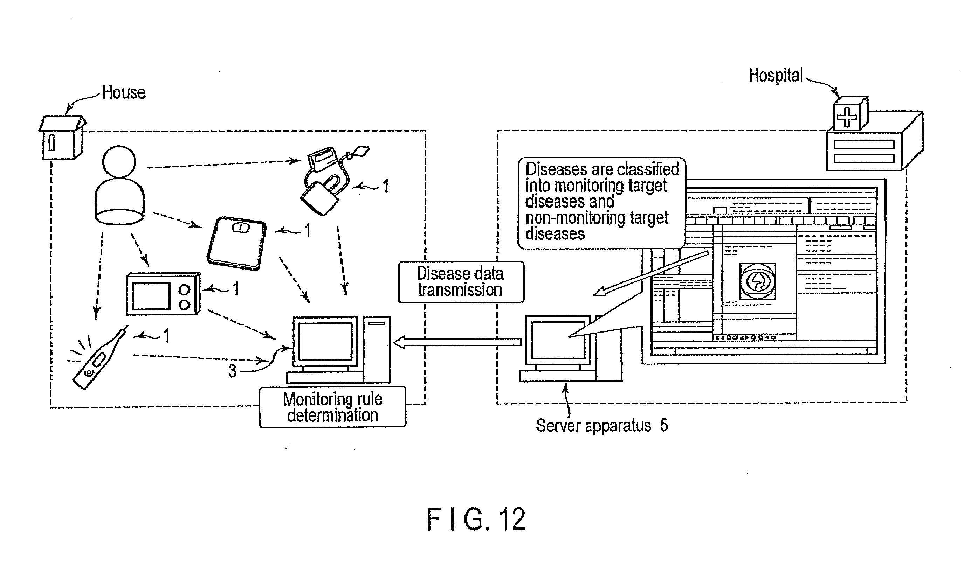

FIG. 12 is a view showing an outline of the healthcare information system according to the third embodiment. As shown in FIG. 12, the healthcare information system according to the third embodiment is typically designed across the house of a monitoring object person and a hospital. The hospital is provided with a server apparatus 5 which manages the medical data of various persons. A client apparatus 3 in the house is connected to the server apparatus 5 in the hospital via, for example a wide area network. The server apparatus 5 classifies the diseases that the monitoring object person has into monitoring target diseases and non-monitoring target diseases, and transmits only the disease data associated with the monitoring target diseases to the client apparatus 3 via the wide area network. In other words, the server apparatus 5 extracts monitoring target diseases from the diseases that the monitoring object person has, and transmits the disease data associated with the extracted monitoring target diseases to the client apparatus 3.

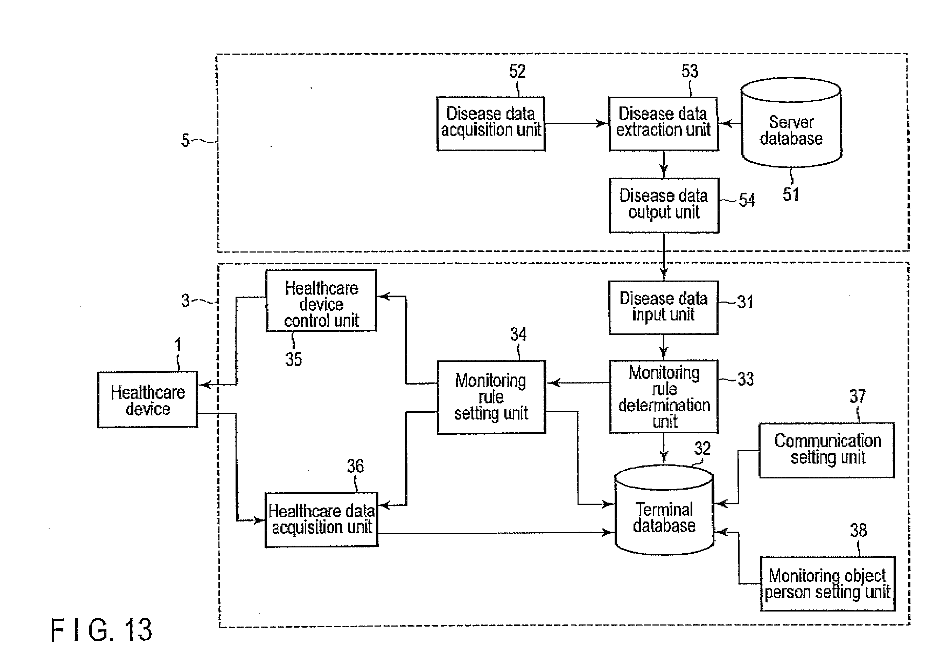

FIG. 13 is a block diagram showing the arrangement of the healthcare information system according to the third embodiment. As shown in FIG. 13, the server apparatus 5 includes a server database 51, a disease data acquisition unit 52, a disease data extraction unit 53, and a disease data output unit 54.

The server database 51 is a database in a healthcare information system such as an electronic medical record system constructed in the hospital. The server database 51 manages the medical data of various persons. For example, the server database 51 stores extraction conditions for monitoring target diseases. More specifically, extraction conditions include information as a guideline for the selection of monitoring target diseases. More specifically, extraction conditions are information about findings and past diagnosis results associated with the monitoring object person. Note that the data which the server database 51 stores are not limited to those included in the database in one hospital, and may be those included in the databases in all the hospitals which the monitoring object person has visited.

The disease data acquisition unit 52 acquires disease data associated with the monitoring object person. For example, the disease data acquisition unit 52 acquires disease data associated with the chronic disease of the monitoring object person and the diseases checked in medical examination. The disease data to be acquired are not limited to the data of the diseases which the monitoring object person has, and include disease data associated with complications and possible intercurrent diseases. The format of disease data may be a standard format such as CCD (Continuity of Care Document), CCR (Continuity of Care Record), or HL7_CDA, or may be a personalized format. HL7_CDA is the clinical document standard of HL7.

The disease data extraction unit 53 acquires the disease data associated with the monitoring object person which is acquired by the disease data acquisition unit 52. The disease data extraction unit 53 also acquires the extraction conditions associated with the acquired disease data (more specifically, disease name data) from the server database 51. The disease data extraction unit 53 then classifies the diseases associated with the disease data into monitoring target diseases and non-monitoring target diseases in accordance with the acquired extraction conditions. In other words, the disease data extraction unit 53 extracts disease data associated with monitoring target diseases from the diseases associated with the disease data.

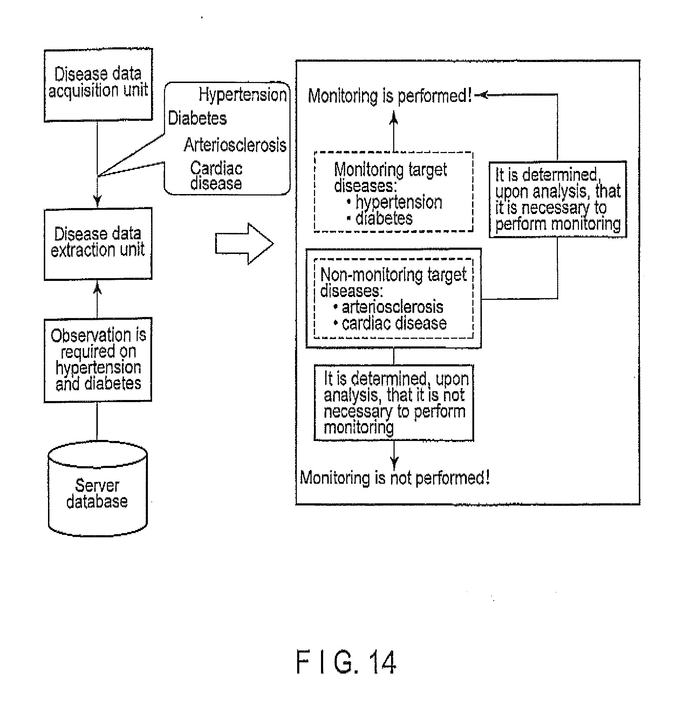

Monitoring target disease extraction processing by the disease data extraction unit 53 will be described below with reference to FIG. 14. FIG. 14 is a block diagram showing a concrete example of extraction processing. As shown in FIG. 14, assume the diseases that the monitoring object person has are hypertension, diabetes, arteriosclerosis, and cardiac disease. Assume also that the extraction conditions are information indicating that "observation is required on hypertension and diabetes". In this case, the disease data extraction unit 53 classifies hypertension and diabetes as monitoring target diseases and arteriosclerosis and cardiac disease as non-monitoring target diseases. Note that if the monitoring object person is a healthy body, the disease data extraction unit 53 classifies all the diseases checked in medical examination as monitoring target diseases. In this manner, the disease data extraction unit 53 extracts monitoring target diseases.

The disease data extraction unit 53 can change a non-monitoring target disease into a monitoring target disease in accordance with the remaining capacity of the memory of the server database 51. If, for example, the remaining capacity of the memory of the server database 51 allows to store healthcare data corresponding to a non-monitoring target disease for the storage period, the disease data extraction unit 53 changes the non-monitoring target disease into a monitoring target disease.

The disease data output unit 54 transmits disease data associated with the monitoring target disease extracted by the disease data extraction unit 53 to the client apparatus 3 (more specifically, the disease data input unit 31) via the wide area network. Thereafter, the client apparatus 3 determines monitoring rules corresponding to the monitoring target diseases, acquires healthcare data in accordance with the monitoring rules, and stores the acquired data in the terminal database 32 in the same manner as in the first embodiment.

With the above arrangement, the healthcare information system according to the third embodiment can extract monitoring target diseases with high priority from the diseases that the monitoring object person has, and acquire only healthcare data corresponding to the monitoring target diseases via the healthcare device 1. The healthcare information system according to the third embodiment can therefore provide monitoring most suitable for the monitoring object person.

As described above, the healthcare information system according to the third embodiment can achieve an improvement in monitoring efficiency.

### (Fourth Embodiment)

It is preferable to periodically check monitoring rules because the health condition of the monitoring object person changes over time. Observing the healthcare data acquired by monitoring allows to follow up treatment and know the health condition of the monitoring object person. A healthcare information system according to the fourth embodiment uses various data stored in the client apparatus as references when the server apparatus acquires disease data. The healthcare information system according to the fourth embodiment will be described below. Note that the same reference numerals in the following description denote constituent elements having almost the same functions as those in the first, second, and third embodiments, and a repetitive description will be made only when required.

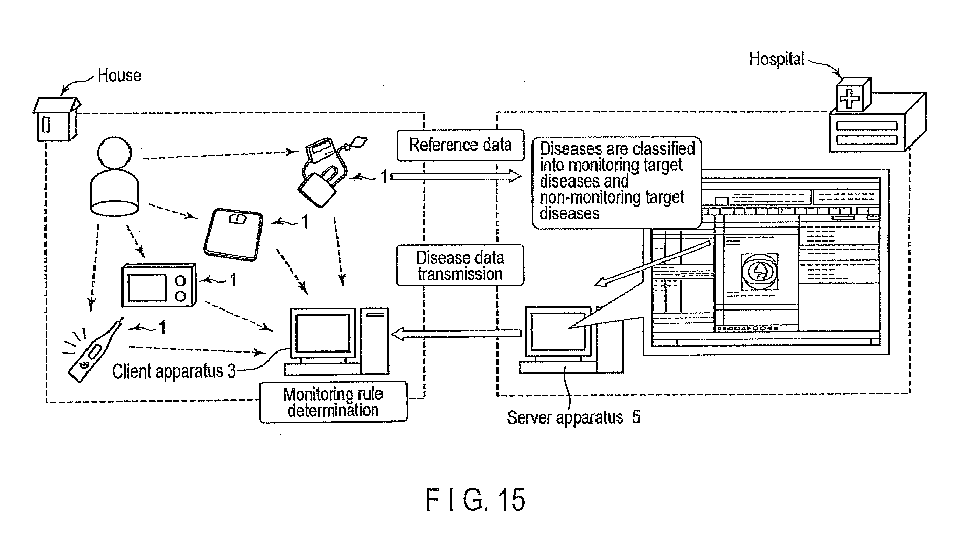

FIG. 15 is a view showing an outline of the healthcare information system according to the fourth embodiment. As shown in FIG. 15, a client apparatus 3 transmits reference data associated with the monitoring object person to a server apparatus 5 to, for example, update monitoring target diseases. Reference data is data obtained by combining the data of a measurement type as a monitoring target with object person data and disease data. Measurement type data, object person data, and disease data which constitute reference data are data which have already been stored in a terminal database 32 of the client apparatus 3. The server apparatus 5 acquires new disease data by using the reference data, extracts disease data associated with the monitoring target diseases from the acquired new disease data, and transmits the extracted disease data to the client apparatus 3.

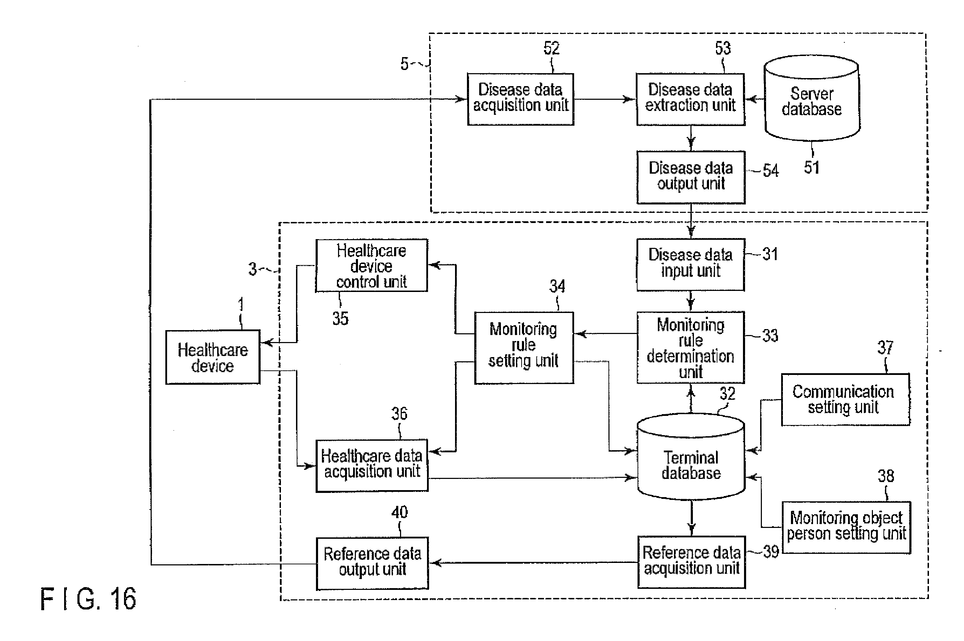

FIG. 16 is a block diagram showing the arrangement of the healthcare information system according to the fourth embodiment. As shown in FIG. 16, the client apparatus 3 according to the fourth embodiment further includes a reference data acquisition unit 39 and a reference data output unit 40.

The reference data acquisition unit 39 reads out the data of a measurement type as a monitoring target from the terminal database 32. The reference data acquisition unit 39 also reads out object person data and disease data associated with the monitoring object person from the terminal database 32. The reference data acquisition unit 39 generates reference data associated with the monitoring object person by combining the readout data of the measurement type with the object person data and the disease data.

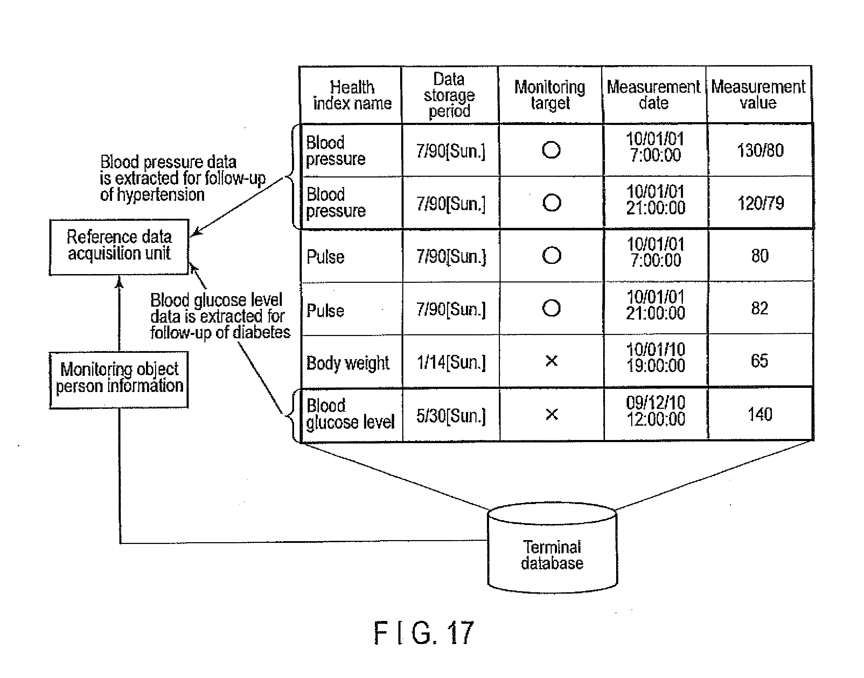

FIG. 17 is a view showing a concrete example of reference data acquisition processing by the reference data acquisition unit 39. As shown in FIG. 17, the terminal database 32 associates the data of measurement types such as blood pressure, pulse, body weight, and blood glucose level with monitoring rules, measurement values (healthcare data), and the like. The reference data acquisition unit 39 reads out object person data and disease data associated with the monitoring object person from the terminal database 32. For example, referring to the medical history or disease data in the object person data allows to know the names of diseases that the monitoring object person has. In the case shown in FIG. 17, assume that the monitoring object person suffers from hypertension and diabetes, and it is necessary to follow up hypertension and diabetes. The reference data acquisition unit 39 reads out the data of blood pressures associated with hypertension and the data of blood glucose levels associated with diabetes from the terminal database 32. The data of blood pressures is not limited to data called "blood pressures", and may include the data of monitoring rules associated with blood pressures and the data of blood pressure values. This applies to the data of other measurement types such as the data of blood glucose levels. The reference data acquisition unit 39 generates reference data associated with the monitoring object person by combining the readout data of blood pressures and the readout data of blood glucose levels with object person data and disease data.

The reference data output unit 40 transmits the reference data acquired by the reference data acquisition unit 39 to a disease data acquisition unit 52 of the server apparatus 5. The disease data acquisition unit 52 acquires disease data based on the reference data. The server apparatus 5 then extracts the disease data of monitoring target diseases from the acquired disease data in the same manner as in the third embodiment. That is, the server apparatus 5 updates monitoring targets by using reference data. The server apparatus 5 transmits the extracted disease data of the monitoring target diseases to the client apparatus 3. The client apparatus 3 acquires healthcare data corresponding to the transmitted monitoring target diseases via the healthcare device 1.

With the above arrangement, the healthcare information system according to the fourth embodiment can update monitoring target diseases by using reference data. It is therefore possible to execute monitoring most suitable for follow-up of treatment and the health condition of the monitoring object person.

As described above, the healthcare information system according to the fourth embodiment can achieve an improvement in monitoring efficiency.

### (Modification)

In the case of acquisition'method 1 in the first embodiment, the terminal database 32 stores healthcare data, of all the healthcare data measured by the healthcare device 1, which match monitoring rules. However, the terminal database 32 may store all the healthcare data measured by the healthcare device 1. In this case, healthcare data which does not match the monitoring rules is deleted at a preset timing. A healthcare information system according to a modification of this embodiment will be described below. Note that the same reference numerals in the following description denote constituent elements having almost the same functions as those in the first, embodiment, and a repetitive description will be made only when required.

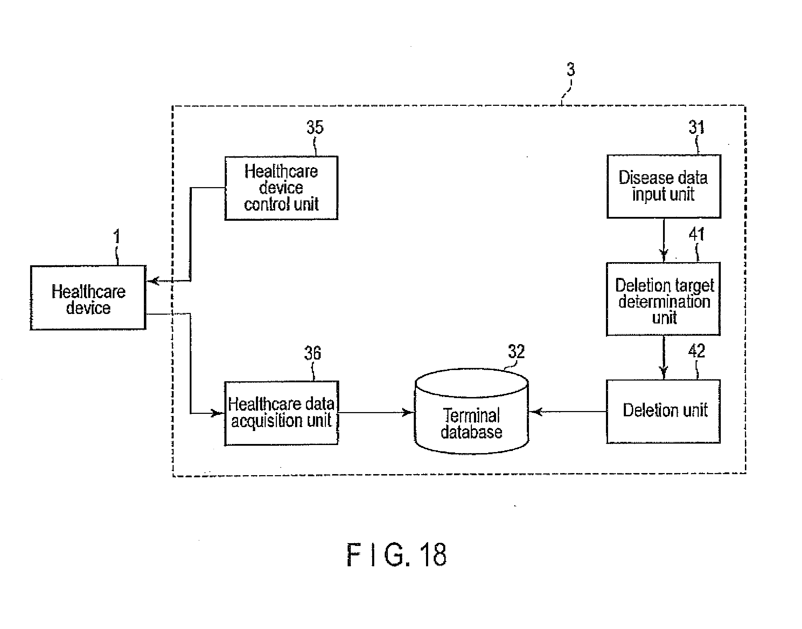

FIG. 18 is a block diagram showing the arrangement of the healthcare information system according to the modification of this embodiment. As shown in FIG. 18, the healthcare information system according to the modification includes the disease data input unit 31, the terminal database 32, the healthcare device control unit 35, the healthcare data acquisition unit 36, a deletion target determination unit 41, and a deletion unit 42.

The deletion target determination unit 41 determines a measurement type as a deletion target from a plurality of measurement types in accordance with the disease names in the disease data input from the disease data input unit 31. More specifically, the deletion target determination unit 41 determines a measurement type as a deletion target by using the monitoring rule table in accordance with the disease names. For example, the deletion target determination unit 41 searches the monitoring rule table by using the disease names as keywords, and determines a disease name which is not associated on the monitoring rule table. The data of measurement values associated with the determined disease name is a deletion target. The deletion target determination unit 41 can determine the deletion timing of data in accordance with disease names. Disease names and deletion timings are associated with each other in a table in advance. The deletion target determination unit 41 determines a deletion timing corresponding to a disease name by referring to this table.

The deletion unit 42 deletes healthcare data, of the healthcare data acquired by the healthcare data acquisition unit 36, which is associated with the disease name determined by the deletion target determination unit 41 from the terminal database 32. The deletion unit 42 deletes healthcare data at the deletion timing determined by the deletion target determination unit 41. Note that the user can arbitrarily set a deletion timing. For example, the user can arbitrarily set a deletion timing such that the deletion unit 42 deletes healthcare data, for example, once a day, at midnight every day, once a week, or at midnight every Monday.

In this manner, the healthcare information system according to this modification can delete healthcare data which is not associated with the disease input by the monitoring object person from the terminal database 32. That is, the healthcare information system can automatically delete healthcare information unnecessary for monitoring, which is stored in the terminal database 32, or allows the user to arbitrarily delete healthcare information unnecessary for monitoring. The data amount of healthcare data stored in the terminal database 32 is small as compared with the prior art in which no healthcare data is deleted. With such reduction in data amount, the data capacity of the terminal database 32 in the healthcare device 1 can be suppressed as compared with the prior art. In addition, it is possible to reduce the complexity of data management.

As described above, the healthcare information system according to this modification can achieve an improve in monitoring efficiency.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. A healthcare information system **characterised by** comprising:
a measurement type storage unit (32) configured to store a plurality of measurement types and healthcare information in association with each other;
an input unit (31) configured to input healthcare information associated with a monitoring object person;
a measurement type determination unit (33) configured to determine a measurement type associated with the input healthcare information in the measurement type storage unit (32);
an acquisition unit (36) configured to acquire a measurement value of the monitoring object person associated with the determined measurement type via a healthcare device; and
a measurement value storage unit (32) configured to store the acquired measurement value.

2. The system of claim 1, **characterized in that** the healthcare information includes at least one of disease information, examination information, medical interview information, and patient information.

3. The system of claim 1, **characterized by** further comprising an monitoring rule determination unit (33) configured to determine a monitoring rule including at least one of a storage period of the measurement value and an acquisition timing in accordance with a medical characteristic of the measurement type,
wherein the acquisition unit (36) acquires a measurement value matching the determined monitoring rule from measurement values from the healthcare device, and
the measurement value storage unit (32) stores the measurement value matching the determined monitoring rule.

4. The system of claim 1, **characterized by** further comprising:
a monitoring rule determination unit (33) configured to determine an monitoring rule including at least one of a storage period of the measurement value and an acquisition timing in accordance with a medical characteristic of the measurement type; and
a control unit (35) configured to control the healthcare device to measure a measurement value in accordance with the determined monitoring rule,
wherein the measurement value storage unit stores a measurement value acquired by the acquisition unit via the controlled healthcare device.

5. The system of claim 1, **characterized by** further comprising a storage period determination unit (33) configured to determine a storage period of the measurement value in accordance with the medical characteristic of the measurement type,
wherein the measurement value storage unit (32) stores the acquired measurement value for only the determined storage period.

6. The system of claim 1, **characterized by** further comprising a storage period determination unit (33) configured to determine a storage period of a measurement value in accordance with a remaining storage capacity of the measurement value storage unit (32),
wherein the measurement value storage unit (32) stores the acquired measurement value for only the determined storage period.

7. The system of claim 1, **characterized by** further comprising an extraction unit (53) configured to extract healthcare information of a monitoring target from the input healthcare information in accordance with at least one of a finding and diagnosis result associated with the monitoring object person,
wherein the measurement type determination unit (33) determines a measurement type associated with the extracted healthcare information in the measurement type storage unit (32).

8. The system of claim 1, **characterized by** further comprising:
a management unit (37) configured to manage a communication setting file including at least one of a transmission data format and a communication standard; and
a setting unit (38) configured to set a communication setting file associated with the healthcare device in the management unit (37).

9. The system of claim 1, **characterized by** further comprising:
a management unit (32) configured to manage an object person file including at least one of an age, sex, and medical history; and
a setting unit (37) configured to set an object person file associated with the monitoring object person in the management unit (32).

10. The system of claim 1, **characterized by** further comprising an output unit (40) configured to output data including at least one of data of healthcare information, data of a measurement value, data of an monitoring rule, and data of a patient file associated with the monitoring object person to a server apparatus (5) via a network.

11. A healthcare information system which monitors a measurement value of a health index of a monitoring object person by using a healthcare device, the system **characterized by** comprising:
a measurement type storage unit (32) configured to store a plurality of measurement types and healthcare information in association with each other;
an input unit (31) configured to input healthcare information associated with the monitoring object person; and
a measurement type determination unit (33) configured to determine a measurement type associated with the input healthcare information in the measurement type storage unit (32).

12. A healthcare information system **characterized by** comprising:
a measurement value storage unit (32) configured to store a plurality of measurement values associated with a plurality of healthcare information;
a measurement type storage unit (32) configured to store a plurality of measurement types and healthcare information in association with each other;
an input unit (31) configured to input healthcare information associated with a monitoring object person;
a determination unit (41) configured to determine a measurement type as a deletion target from the measurement types in accordance with the input healthcare information; and
a deletion (42) unit configured to delete a measurement value associated with the determined measurement type from the measurement value storage unit.

13. The system of claim 12, **characterized in that** the determination unit (41) determines a deletion timing in accordance with the input healthcare information, and
the deletion unit (42) deletes a measurement value associated with the determined measurement type from the measurement value storage unit (32) at the determined deletion timing.
